# EUROPEAN PATENT APPLICATION

(11) **EP 2 878 596 A1**
(43) Date of publication of application: **03.06.2015**
(21) Application number: 13195067.7
(22) Date of filing: 29.11.2013
(51) Int. Cl.: C07D 295/096

(54) **Synthesis of vortioxetine via (2-(piperazine-1-yl)phenyl)lithium intermediates**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention provides a new synthetic process for the production of 1-(2-((2,4-dimethylphenyl)thio)phenyl)piperazine (vortioxetine), a drug for the treatment of depression and anxiety, which is conducted via (2-(piperazine-1-yl)phenyl)lithium intermediates.

## Description

### Field of the Invention

This invention relates to a new and advantageous process for the synthesis of 1-(2-((2,4-dimethylphenyl)thio)phenyl)piperazine (vortioxetine) a drug for the treatment of depression and anxiety.

### Background of the Invention

Vortioxetine is disclosed as Example 1e in WO 2003/029232 A1 and is described as being prepared analogously to Example 1. The process used to prepare Example 1 involves the preparation of 1-(2-((2-(trifluoromethyl)phenyl)thio)phenyl)piperazine on a solid polystyrene support, followed by decomplexation using visible light irradiation, and purification by preparative LC-MS and ion-exchange chromatography. The overall yield for the preparation of vortioxetine is described as 17%.

Several alternative palladium catalyzed processes for the preparation of vortioxetine are described in Examples 17 to 25 of WO 2007/144005 A1. These processes describe the preparation of vortioxetine from 2,4-dimethylthiophenol and 2-bromoiodobenzene (or 1,2-dibromobenzene) starting materials *via* a 1-(2-bromo-phenylsulfanyl)-2,4-dimethyl-benzene intermediate. Each of these processes involves the use of a palladium catalyst and a phosphine ligand.

The preparation of vortioxetine is also described by Bang-Andersen et al. in J. Med. Chem. (2011), Vol. 54, 3206-3221. Here, in a first step, *tert*-butyl 4-(2-bromophenyl)piperazine-1-carboxylate intermediate is prepared from Boc-piperazine and 2-bromoiodobenzene in a palladium catalyzed coupling reaction. *tert*-Butyl 4-(2-bromophenyl)piperazine-1-carboxylate is then reacted with 2,4-dimethylthiophenol, again in the presence of palladium catalyst and a phosphine ligand, to provide Boc-protected vortioxetine. In the final step, vortioxetine is deprotected using hydrochloric acid to give vortioxetine hydrochloride.

WO 2013/102573 A1 describes a reaction between 1-halogen-2,4-dimethyl-phenyl, 2-halogenthiophenol and an optionally protected piperazine in the presence of a base and a palladium catalyst consisting of a palladium source and a phosphine ligand.

Each of the above processes has disadvantages. The process described in WO 2003/029232 is low yielding and unsuitable for the large scale production of vortioxetine, whereas the processes described in WO 2007/144005 A1, WO 2013/102573 A1 and by Bang-Andersen *et al.* require the use of expensive starting materials, palladium catalyst and phosphine ligand. In addition, the toxicity of palladium is well known, Liu et al. Toxicity of Palladium, Toxicology Letters, 4 (1979) 469-473, and the European Medicines Agency' s Guideline on the Specification for Residues of Metal Catalysts sets clear limits on the permitted daily exposure to palladium arising from palladium residue within drug substances, www.ema.europa.eu. Thus it would be desirable to avoid the use of a palladium catalyst in the synthesis of vortioxetine and the subsequent purification steps required to remove palladium residue from the final pharmaceutical product.

### Summary of the Invention

Various aspects, advantageous features and preferred embodiments of the present invention as summarized in the following items, respectively alone or in combination, contribute to solving the object of the invention.
(1) A process for the manufacture of a compound of formula **IVa,** or a salt thereof, 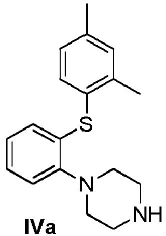 said process comprising the steps of
   a) reacting a compound of formula **I** 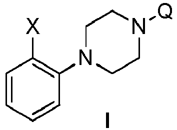 wherein X represents hydrogen or a halogen, and Q represents hydrogen, an amino protecting group, or the 2,4-dimethylphenylsulfide moiety of formula 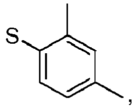 which 2,4-dimethylphenylsulfide moiety is coupled to the nitrogen of formula **I** via the sulphur atom,
      by lithiation, to give a compound of formula **II** or **II'** 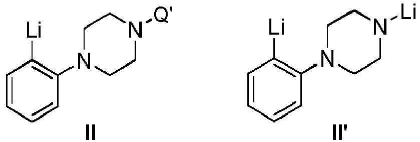 wherein Q' represents said amino protecting group, or the 2,4-dimethylphenylsulfide moiety, and
   b) converting the compound of formula **II** or **II'** into the compound of formula **IVa,**
   c) optionally, converting the obtained compound of formula **IVa** into the salt thereof.
   The term "amino protecting group", Pg, as employed herein means any group that is used for protection of amines. Typically, such group is selected from the group consisting of *tert*-butyloxycarbonyl (Boc), benzyloxycarbonyl (Cbz), fluorenylmethoxycarbonyl (Fmoc), unsubstituted or substituted benzyl, or benzenesulfonyl (Bs), p-toluenesulfonyl (Ts), 2-naphthylsulfonyl, trimethylsilyl (TMS), trifluoroacetyl (TFA), trityl (Tr), trichloroacetyl (TCA), formyl (CHO), acetyl (Ac), benzoyl (Bz), C₄-C₅-*tert*-alkyl, preferably *tert*-butyl (*t*-Bu). Preferably, the Pg is selected from the group consisting of triphenylmethyl (trityl, Tr), *tert-*butoxycarbonyl (Boc) and trimethylsilyl group (TMS).
   The term "lithiation" as used herein means any process that is used to introduce lithium into the molecule. Typically, lithium is introduced by implementing alkyl lithium or lithium amide reagents, preferably C₁-C₁₀ alkyl lithium reagents, most preferably n-, s-, or t-butyllithium (BuLi).
(2) The process according to item 1, wherein the halogen is Br.
(3) The process according to item 1 or item 2, wherein the amino protecting group is triphenylmethyl, *tert*-butoxycarbonyl or trimethylsilyl.
(4) The process according to any of the previous items, wherein the lithiation is conducted with n-, s- or t-butyllithium in an aprotic solvent.
   The term "aprotic solvent" as used herein means a solvent incapable of acting as a proton donor, for example, a solvent selected from the group consisting of ethers and alkanes. The preferred ethers used are diethylether and tetrahydrofurane (THF). The preferred alkanes are hexane and cyclohexane.
(5) The process according to item 4, wherein the aprotic solvent is an ether or an alkane.
(6) The process according to item 5, wherein the ether is diethylether or THF, preferably THF.
(7) The process according to item 5, wherein the alkane is hexane or cyclohexane.
(8) The process according to item 1, wherein the compound of formula **II** or **II'** is not isolated.
(9) The process according to item 1, wherein Q in the compound of formula **I** represents hydrogen, and step b) is conducted by introducing the 2,4-dimethylphenylsulfide moiety to the compound of formula **II'** to give a compound of formula **IVa.**
(10) The process according to item 1, wherein Q in the compound of formula **I** represents an amino protecting group, and step b) is conducted by introducing the 2,4-dimethylphenylsulfide moiety to the compound of formula **II** to give a compound of formula **IVb** 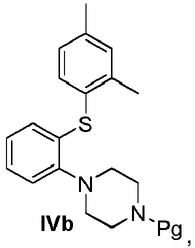 and, deprotecting the protected amino group to obtain the compound of formula **IVa.**
(11) The process according to item 10, wherein the amino group is protected with the triphenylmethyl group or the *tert*-butoxycarbonyl group.
(12) The process according to claim 10, wherein the amino group is protected with the trimethylsilyl group.
(13) The process according to item 10 and 11, wherein the deprotection step is conducted in acidic conditions, preferably with HBr or HCl.
(14) The process according to item 9 or item 10, wherein the 2,4-dimethylphenylsulfide moiety is introduced using 1,2-bis(2,4-dimethylphenyl)disulfane or 2,4-dimethylphenylsulfenyl halide, preferably 2,4-dimethylphenyl hypochlorothioite (2,4-dimethylphenylsulfenyl chloride).
(15) The process according to item 1, wherein Q in the compound of formula **I** represents the 2,4-dimethylphenylsulfide moiety of formula 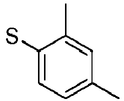 wherein said moiety is coupled to the nitrogen via the sulphur atom, and step b) is conducted by the re-arrangement of the 2,4-dimethylphenylsulfide moiety to give the compound of formula **IVa.**
(16) The process according to item 15, wherein the lithiation temperature is from -100°C to -50°C, preferably the lithiation temperature is about -80°C.
   The term "about" means approximately, in the region of, roughly, or around. When the term is used in conjunction with a numerical range, it modifies that range by extending the boundaries above and below the numerical values set forth. In general, the term "about" is used herein to modify a numerical value above and below the stated value by a variance of 10%.
(17) The process according to item 15 and 16, wherein the re-arrangement of the 2,4-dimethylphenylsulfide moiety is conducted at a temperature of from 0°C to 50°C, preferably at room temperature, in an aprotic solvent.
(18) The process according to item 17, wherein the aprotic solvent is ether, preferably THF.
(19) The process according to item 15, wherein the compound of formula **I**, wherein Q represents the 2,4-dimethylphenylsulfide moiety of formula 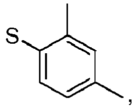 is prepared by a process comprising the steps of
   a) reacting 2,4-dimethylbenzenethiol with trichloroisocyanuric acid to give 2,4-dimethylphenyl hypochlorothioite, and
   b) reacting the 2,4-dimethylphenyl hypochlorothioite with the compound of formula **I"** 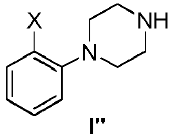 wherein X represents hydrogen or halogen.
(20) The process according to item 19, wherein the 2,4-dimethylphenyl hypochlorothioite is not isolated.
(21) The process according to item 1, wherein the salt of the compound of formula **IVa** is a hydrobromide salt or a hydrochloride salt.
(22) A compound of formula **I'** 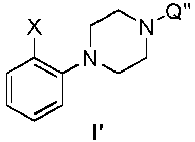 wherein X represents hydrogen or a halogen and Q" represents triphenylmethyl or 2,4-dimethylphenylsulfide.
(23) A compound of formula **IVb'** 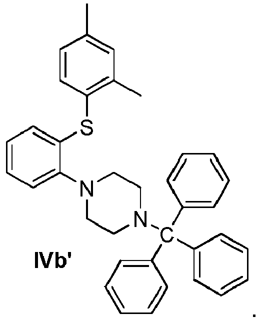
(24) A compound of formula **Ic, Id, Ig,** or **Ih** 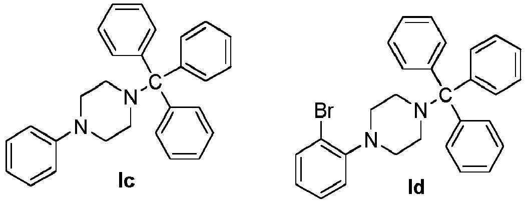 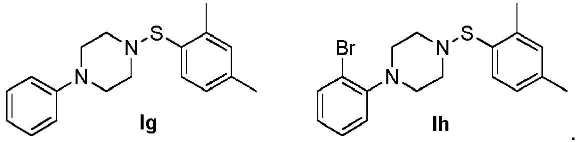
(25) Use of the compounds as defined in any one of item 22 to 24 in the preparation of vortioxetine or a salt thereof.
(26) A process for the preparation of a pharmaceutical composition comprising a compound of formula **IVa,** or a salt thereof 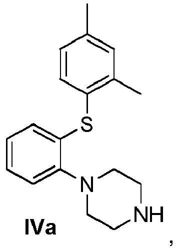 comprising the steps of:
   a) preparing a compound of formula **IVa,** or a salt thereof, by carrying out the process according to any one of items 1 to 21, and
   b) mixing the compound of formula **IVa,** or a salt thereof, with a pharmaceutically acceptable carrier and/or excipient.

The new process defined above provides vortioxetine, or salts thereof, in a high yield without the need of using the palladium catalyst and phosphine ligand, nor expensive starting materials, which provides an industrially applicable, environmentally friendly and economically improved process for obtaining vortioxetine, or salts thereof. The present invention further provides new intermediate compounds that are highly useful as key intermediates in the preparation of vortioxetine or salts thereof. The intermediates are crystalline, suitable for optional purification by recrystallization.

### Detailed Description of the Invention

The invention is described below in further detail by embodiments, without being limited thereto.

A general concept of the process of the present invention may be represented in Scheme 1. Compound **I** is subjected to the lithiation reaction to form a (2-(piperazin-1-yl)phenyl)lithium intermediate **II** or **II'**. Any known lithiation method can be used for this step, for example, alkyl lithium or lithium amide reagents can be used, preferably C₁-C₁₀ alkyl lithium reagents, most preferably n-, s-, or t-butyllithium (BuLi) in an aprotic solvent is used. In the next step, the (2-(piperazin-1-yl)phenyl)lithium intermediate **II** is converted either to the compound of formula **IVb,** if Q in the compound of Formula **I** is the amino protecting group Pg, or directly to vortioxetine **(IVa),** if Q in the compound of Formula **I** is hydrogen or the 2,4-dimethylphenylsulfide moiety of formula

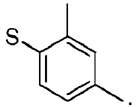

In case Q in the compound of formula **I** is hydrogen or an amino protecting group, the compound of formula **IVa** or **IVb,** respectively, is formed by the reaction of substitution, wherein the 2,4-dimethylphenylsulfide moiety is introduced using 1,2-bis(2,4-dimethylphenyl)disulfane **(IIIa)** or 2,4-dimethylphenylsulfenyl halide, preferably 2,4-dimethylphenyl hypochlorothioite **(IIIb).** In case the Pg-protected compound of formula **IVb** is formed, a deprotection step is needed to convert it to the compound of formula **IVa** (vortioxetine).

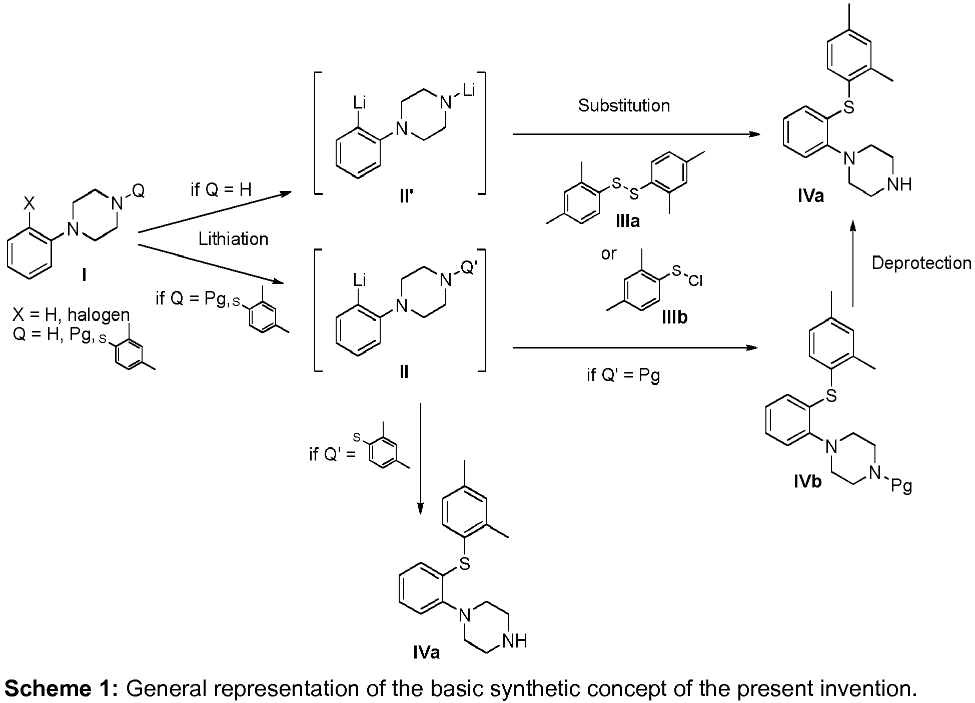

Optionally, the vortioxetine compound obtained by the process of the present invention can also be converted to a salt thereof, for example to a hydrobromide salt **(IVa.HBr)** or a hydrochloride salt **(IVa.HCl).**

According to a preferred embodiment, the lithium compound **II** or **II'** is not isolated and reacts with an appropriate electrophile, which can be included on the molecule **I** itself, if Q in the compound of formula **I** is the 2,4-dimethylphenylsulfide moiety, or is added during the substitution step as described above and in more detail in the specific embodiments of vortioxetine synthesis that follow.

In a specific embodiment presented in Scheme 2, 1-phenylpiperazine **(Ia)** or 1-(2-bromophenyl)piperazine **(Ib),** respectively, is used as the starting compound, which is transformed to the *ortho*-lithiated compound **II'** by using standard lithiated procedures, preferably using n-, s-, or t-BuLi in aprotic solvent, preferably ethers and alkanes. The most preferable ethers used are diethyl ether and tetrahydrofuran (THF), while the most preferable alkanes used are hexane and cyclohexane. Lithiation occurs at an appropriate temperature, depending on the compound, the solvent, and the lithiating agent used. If, for example, s-BuLi in cyclohexane is used to lithiate the compound **Ia,** the lithiation may occur, for example, at 50°C (see Example 1). If, in another example s-BuLi in THF is used to lithiate the compound **Ib,** the lithiation may occur, for example, at -78°C (see Example 2). In the next step the 2,4-dimethylphenylsulfide moiety can be introduced by the reaction of substitution by adding the 1,2-bis(2,4-dimethylphenyl)disulfane **(IIIa)** or 2,4-dimethylphenyl hypochlorothioite **(IIIb),** respectively, as the electrophiles into the reaction pot. The substitution can be conducted in an aprotic solvent, for example in THF. The obtained compound of formula **IVa** (vortioxetine) can be purified. Optionally, **IVa** can also be converted to a salt thereof.

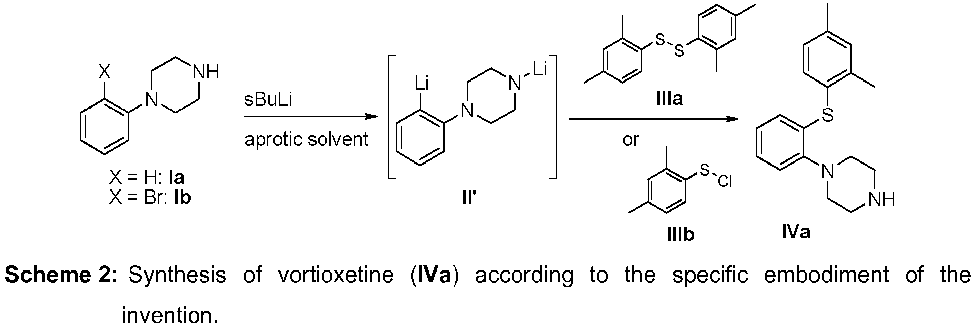

In a further specific embodiment of the present invention shown in Scheme 3, the starting compound is selected from the group consisting of the N-trityl **(Ic** and **Id)** and N-Boc **(Ie** and **If)** protected compounds, which are prepared from the corresponding compounds of formula **I,** wherein Q is hydrogen **(Ia** and **Ib).** The introduction of the amino protecting group is performed in the presence of a base, for example, the base can be selected from the group consisting of tertiary amines, pyridine, carbonates, phosphates. The preferred base is triethylamine. Lithiation and introduction of 2,4-dimethylphenylsulfide moiety may occur under the same reaction conditions as described above. First, lithiation is conducted using an appropriate lithiation agent, preferably n-, s-, or t-BuLi in an aprotic solvent. The substitution preferably occurs by using the compound **IIIa** or **IIIb,** respectively, to give compounds **IVb'** or **IVb",** depending on the protecting group used.

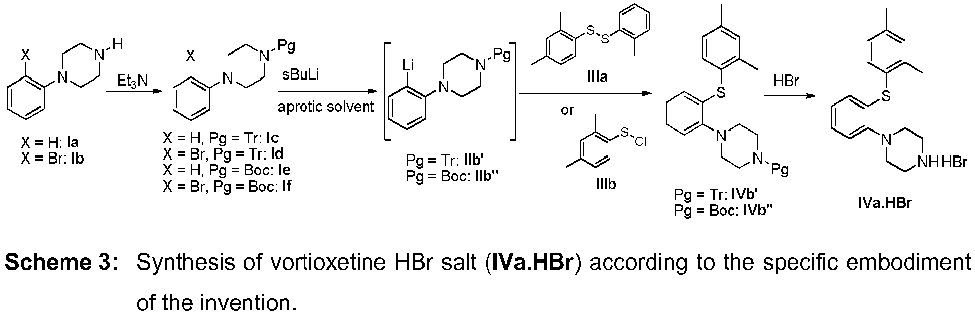

Due to the presence of the amino protecting group, the deprotection step is needed to obtain the compound of formula **IVa.** The deprotection conditions depend on the nature of the respective protecting group. For example, in case of N-Tr and N-Boc protecting groups, acidic conditions are used. If for instance HBr in any acid resistible solvent is used for the deprotection step, the vortioxetine HBr salt **(IVa.HBr)** is obtained in one step. Similarly, if HCl is used, the vortioxetine HCl salt is obtained.

In a further specific embodiment of the present invention presented in Scheme 4, the 1-phenylpiperazine **(Ia)** is protected using a silyl amino protecting group, preferably trimethylsilyl (TMS) to give the compound **Ig,** which is preferably not isolated. In the next step lithiation is conducted using n-, s- or t-BuLi in an aprotic solvent. The substitution is conducted using a compound **IIIa** or **IIIb,** respectively, to give the compound **IVa.** In this case the deprotection step is conducted during the isolation of **IVa,** as the TMS protecting group is readily removed, for example by washing the TMS-protected compound of formula **IVa (IVb"')** with water.

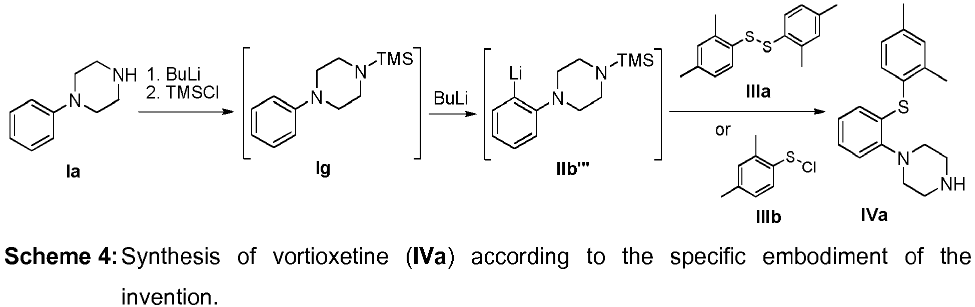

In a further specific and preferred embodiment shown in Scheme 5, the process of the present invention starts from 2,4-dimethylbenzenethiol, which is transformed to 2,4-dimethylphenyl hypochlorothioite **(IIIb). IIIb** in the next step reacts in the presence of a base, preferably triethylamine, with 1-phenylpiperazine **(Ia)** or 1-(2-bromophenyl)piperazine **(Ib)** to afford 1-((2,4-dimethylphenyl)thio)-4-phenylpiperazine **(Ig)** or 1-(2-bromophenyl)-4-((2,4-dimethylphenyl)thio)-piperazine **(Ih)** as a white solid. Compounds **Ig** and **Ih** are then lithiated using BuLi, preferably using n- or s-BuLi, at low temperatures, preferably at -100 to -50°C, most preferably at about -80°C, in aprotic solvents, preferably in ethers or alkanes, most preferably in tetrahydrofuran. This is followed by elevating the temperature to the range from 0°C to 50°C, preferably to room temperature, in order to accomplish the re-arrangement of the 2,4-dimethylphenylsulfide group, to give vortioxetine **(IVa).**

The reaction from 2,4-dimethylbenzenethiol to the compound **Ig** or **Ih** can also be made according to one-pot methodologies. In the first step **IIIb** is formed using trichloroisocyanuric acid **(TCCA)** in EtOAc at 0°C. After 30 min Et₃N is added, followed by compound **Ia** or **Ib** to give intermediates **Ig** or **Ih.**

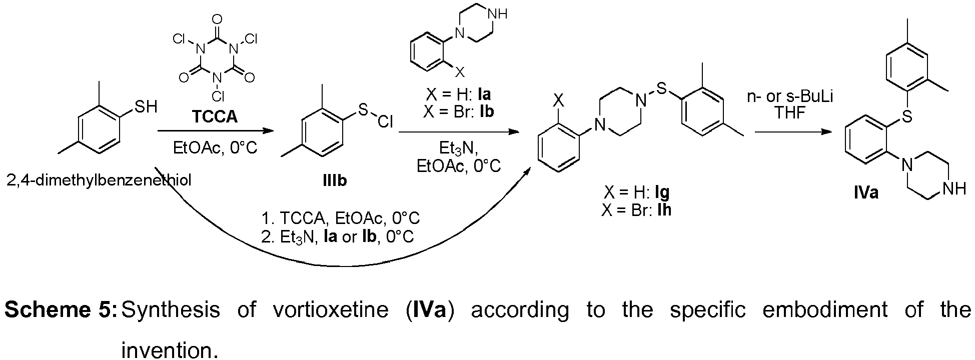

The vortioxetine compound, or a salt thereof, prepared according to the invention may be used or administered on its own, preferably it is administered as a pharmaceutical composition comprising vortioxetine, or a salt thereof, and a pharmaceutically acceptable excipient and/or carrier. Further, the vortioxetine compound, or a salt thereof, prepared according to the invention may be combined with other drugs, especially with drugs used for the treatment of depression and anxiety.

In a further aspect of the present invention, a pharmaceutical composition comprising the compound of formula **IVa** (vortioxetine), or a salt thereof, is prepared by comprising the steps of preparing the compound of formula **IVa,** or a salt thereof, as described above, and mixing the compound of formula **IVa,** or a salt thereof, with a pharmaceutically acceptable carrier and/or excipient. The administration form can be suitably chosen, e.g. a form suitable for oral, parenteral, rectal administration and/or administration by inhalation, and the dosage form may be solid, liquid, or powdery. Therefore, the pharmaceutical composition comprising vortioxetine, or a salt thereof, prepared according to the invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the compound or a finely divided form thereof, or particles comprising the compound, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The compound or a finely divided form thereof, or particles containing the same may be coated by another substance. The powder mixture or particles containing the compound may also be dispensed into capsules.

The pharmaceutical composition comprising vortioxetine, or a salt thereof, prepared according to the invention in a desired dose is generally suitable to treat a disease or condition of a patient in need thereof, speciffically to display a desired activity when treating the depression and anxiety.

Further embodiment of the present invention resides in the provision of valuable intermediate compounds useful in the synthesis of a compound of formula **IVa** (vortioxetine). In particular, the compounds of formula **I'** 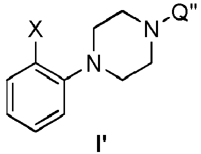 wherein X represents hydrogen or a halogen and Q" represents triphenylmethyl or 2,4-dimethylphenylsulfide; and
the compound of formula **IVb'**

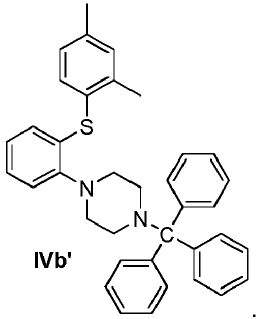

The preferred intermediate compounds useful in the synthesis of a compound of formula **IVa** (vortioxetine) are **Ic, Id, Ig, Ih** and **IVb'**

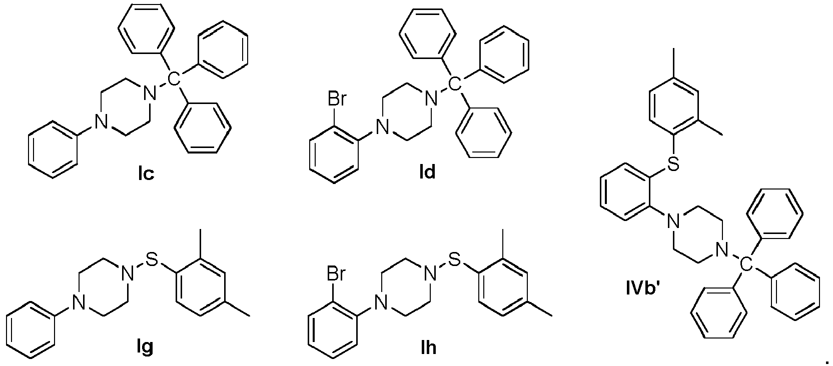

The following Examples are non-limiting and serve to illustrate the invention.

### Experimental Procedures

### Example 1: Preparation of 1-(2-((2,4-dimethylphenyl)thio)phenyl)piperazine (vortioxetine, IVa) from 1-phenylpiperazine (Ia)

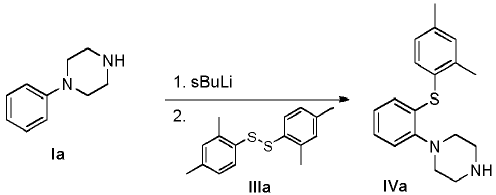

To a solution of **Ia** (0.5 g, 3.08 mmol) in dry cyclohexane (10 mL) while stirring at 25°C was slowly added s-BuLi (6.78 mmol). Obtained mixture was stirred at 50°C for 16 h, then it was cooled to room temperature, and solution of **IIIa** (1.27 g, 4.62 mmol) in dry THF (10 mL) was added. After stirring for 24 h 10 mL water and 10 mL EtOAc were added. Organic layer is then washed with brine (2 x 20 mL), dried over Na₂SO₄, and solvent is evaporated to give crude product, which is purified by chromatography to give title compound. ¹H NMR (CDCl₃, 500 MHz) *δ* 1.63 (br s, 1 H), 2.33 (s, 3H), 2.37 (s, 3H), 3.02-3.09 (m, 8H), 6.52 (m, 1 H), 6.87 (m, 1 H), 7.04 (m, 1 H), 7.06-7.10 (m, 2H), 7.16 (m, 1 H), 7.39 (d, J = 7.8 Hz, 1 H); MS (ESI) m/z: 299 [MH]⁺.

### Example 2: Preparation of 1-(2-((2,4-dimethylphenyl)thio)phenyl)piperazine hydrobromide (vortioxetine HBr, IVa.HBr) from 1-(2-bromophenyl)piperazine (Ib)

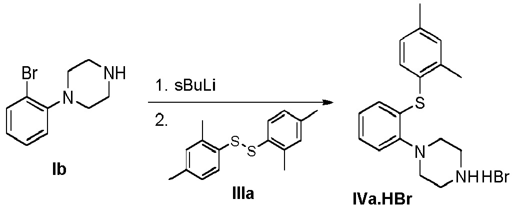

To a solution of **Ib** (0.71 g, 2.93 mmol) in dry THF (10 mL) sBuLi (6.44 mmol) was added at-78°C. After stirring for 15 min, solution of **IIIb** (0.96 g, 3.52 mmol) in dry THF (5 mL) was added at -78°C. Obtained reaction mixture was then stirred at room temperature for 17 h then 10 mL water and 20 mL EtOAc were added. Water layer was separated and organic layer was washed with brine (2 x 20 mL), dried over Na₂SO₄, and solvent was evaporated to give crude product, which was dissolved in iPrOAc (10 mL). 48% HBr (0.33 mL) was then added and obtained mixture was stirred at room temperature for 1 h. Precipitate was filtered off, washed with iPrOAc (2 x 10 mL) and dried to give title compound as off-white powder (0.78 g, 70% yield): ¹H NMR (DMSO-*d₆*, 500 MHz) *δ* 2.23 (s, 3H), 2.32 (s, 3H), 3.15-3.27 (m, 8H), 6.40 (m, 1 H), 6.96 (m, 1 H), 7.08-7.17 (m, 3H), 7.24 (m, 1 H), 7.32 (d, J = 7.8 Hz, 1 H), 8.85 (br, 2H).

### Example 3: Preparation of 1-phenyl-4-tritylpiperazine (Ic)

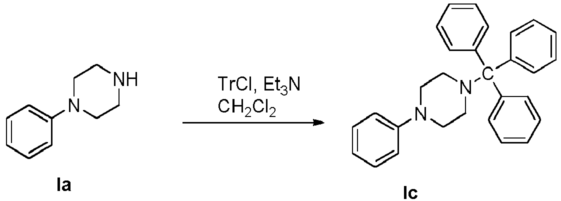

To a solution of 1-phenylpiperazine **Ia** (5.0 g, 30.8 mmol), Et₃N (5.15 mL, 37.0 mmol) in CH₂Cl₂ (30 mL) TrCl (9.0 g, 32.4 mmol) was added. After stirring for 2 h at room temperature water (100 mL) was added and product was extracted to CH₂Cl₂ (2 x 30 mL). Combined organic layers were dried over Na₂SO₄, and solvent was evaporated. To the residue MeOH (50 mL) was added, and obtained mixture was stirred at room temperature. White precipitate was then filtered off, washed with MeOH (2 x 25 mL) and dried to afford title compound **Ic** as white powder (11.72 g, 94% yield): DSC (onset): 173°C; ¹H NMR (CDCl₃, 500 MHz) *δ* 2.07-2.91 (br m, 4H), 3.35 (m, 4H), 6.87 (m, 1 H), 6.93 (m, 2H), 7.20 (m, 3H), 7.25-7.34 (m, 8H), 7.56 (m, 6H); MS (ESI) m/z: 405 [MH]⁺.

### Example 4: Preparation of 1-(2-bromophenyl)-4-tritylpiperazine (Id)

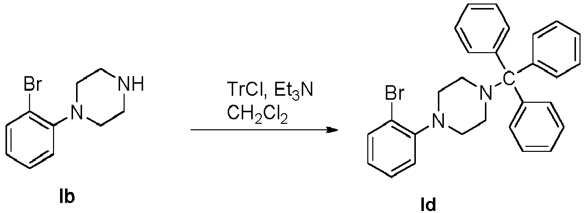

To a solution of 1-(2-bromophenyl)piperazine **Ib** (7.43 g, 30.8 mmol), Et₃N (6.44 mL, 46.2 mmol) in CH₂Cl₂ (40 mL) TrCl (9.0 g, 32.4 mmol) was added. After stirring for 1 h at room temperature 50 mL of saturated aqueous NaHCO₃ solution was added. After stirring for 15 min organic phase was separated, dried over Na₂SO₄, and solvent was evaporated. To the residue MeOH (50 mL) was added, and obtained mixture was stirred at room temperature. After cooled to-18°C, white precipitate was filtered off, washed with MeOH (2 x 20 mL) and dried to afford title compound **Id** as white powder (14.5 g, 97% yield): DSC (onset): 225°C; ¹H NMR (CDCl₃, 500 MHz) *δ* 3.23 (br m, 8H), 6.91 (m, 1 H), 7.16-7.22 (m, 4H), 7.28-7.33 (m, 7H), 7.51-7.60 (m, 7H); MS (ESI) m/z: 484 [MH]⁺.

### Example 5: Preparation of 1-(2-((2,4-dimethylphenyl)thio)phenyl)-4-tritylpiperazine (IIa) from 1-phenyl-4-tritylpiperazine (Ic)

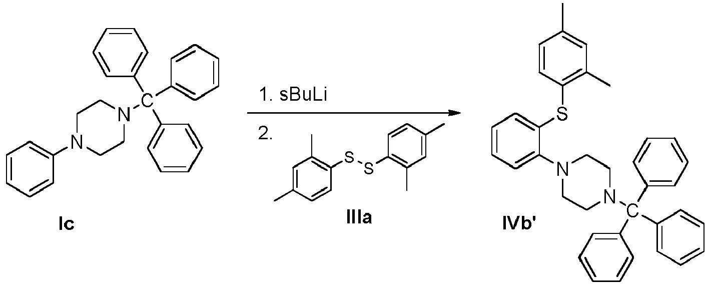

To a solution of **Ic** (0.84 g, 2.07 mmol) in dry Et₂O (10 mL) and dry THF (5 mL) while stirring at 25°C was slowly added s-BuLi (2.17 mmol). Obtained mixture was stirred at room temperature for 1.5 h then a solution of **IIIa** (0.74 g, 2.69 mmol) in dry THF (5 mL) was added. After stirring for 3 days at room temperature 10 mL water and 10 mL EtOAc were added. Organic layer is then washed with brine (2 x 20 mL), dried over Na₂SO₄, and solvent is evaporated to give crude product, which is purified by chromatography to give title compound. MS (ESI) m/z: 541 [MH]⁺.

### Example 6: Preparation of 1-(2-((2,4-dimethylphenyl)thio)phenyl)-4-tritylpiperazine (IIa) from 1-(2-bromophenyl)-4-tritylpiperazine (Id)

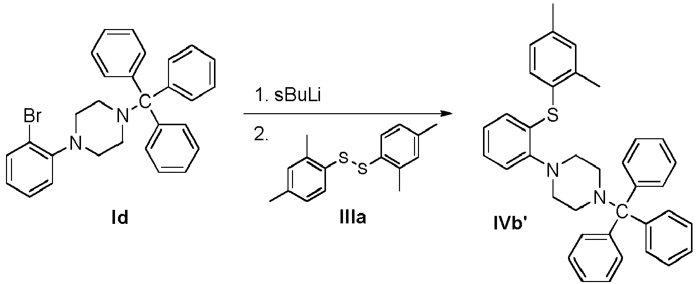

To an ice-cold solution of **Id** (1.0 g, 2.07 mmol) in dry THF (10 mL) was added s-BuLi (2.17 mmol). Obtained reaction mixture was stirred at 0°C for 15 min then solution of **IIIa** (0.74 g, 2.69 g) in dry THF (5 mL) was added. After stirring for 3 days at room temperature water (25 mL) and EtOAc (15 mL) were added. Water layer was separated and washed with EtOAc (20 mL). Combined organic layers were dried over Na₂SO₄, salts were filtered off and filtrate was concentrated to afford yellow oil, which was crystalized from CH₂Cl₂/MeOH mixture to afford title compound as a white powder (0.85 g, 76% yield): MS (ESI) m/z: 541 [MH]⁺.

### Example 7: Preparation of tert-butyl 4-(2-((2,4-dimethylphenyl)thio)phenyl)piperazine-1-carboxylate (IIb) from tert-butyl 4-(2-bromophenyl)piperazine-1-carboxylate (If)

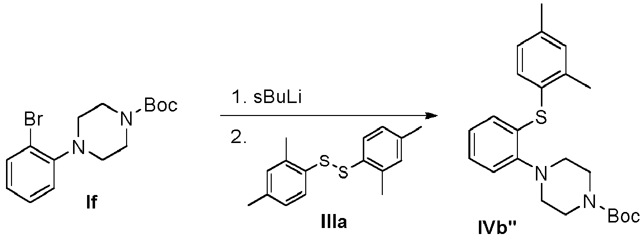

To a solution of *tert*-butyl 4-(2-bromophenyl)piperazine-1-carboxylate **If** (1.0 g, 2.93 mmol) in dry THF (10 mL) sBuLi (2.69 mL, 3.22 mmol) was added at -78°C. After stirring for 15 min, solution of **IIIa** (0.96 g, 3.52 mmol) in dry THF (5 mL) was added at -78°C. Obtained reaction mixture was then stirred at room temperature for 18 h, then 20 mL water was added and product was extracted to EtOAc (2 x 10 mL). Combined organic layers were washed with brine (2 x 20 mL), dried over MgSO₄, and solvent was evaporated to give crude product, which was purified by chromatography (methylcyclohexane/EtOAc) to afford title compound IIb as yellowish oil, which crystalized upon standing (0.96 g, 83% yield): ¹H NMR (CDCl₃, 500 MHz) *δ* 1.51 (s, 9H), 2.33 (s, 3H), 2.37 (s, 3H), 3.02 (m, 4H), 3.63 (m, 4H), 6.53 (dd, J = 1.4, 7.9 Hz, 1 H), 6.88 (m, 1 H), 7.02-7.05 (m, 2H), 7.08 (m, 1 H), 7.16 (m, 1 H), 7.38 (d, J = 7.8 Hz, 1 H); MS (ESI) m/z: 399 [MH]⁺.

### Example 8: Preparation of 1-((2,4-dimethylphenyl)thio)-4-phenylpiperazine (Ig)

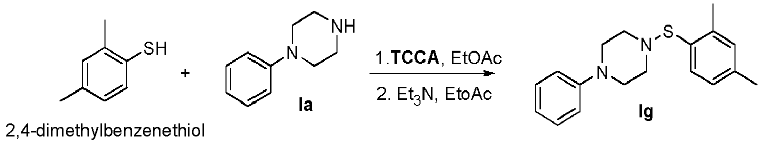

To a solution of trichloroisocyanuric acid **(TCCA)** (2.24 g, 9.65 mmol) in EtOAc (50 mL) was slowly added 2,4-dimethylbenzenethiol (0.49 mL, 3.62 mmol). Obtained reaction mixture was stirred at room temperature for 30 min, then Et₃N (10.1 mL, 72.3 mmol) and solution of **Ia** (4.46 g, 27.5 mmol) in EtOAc (50 mL) was added. After stirring at room temperature for 2 h, salts were filtered off, washed with EtOAc (2 x 20 mL), and filtrate was concentrated. To the remaining MeOH (100 mL) was added and the obtained mixture was stirred at room temperature for 1 h. White precipitate was filtered off, washed with MeOH (2 x 20 mL) and dried to afford title compound as white powder (6.17 g, 75% yield): ¹H NMR (CDCl₃, 500 MHz) *δ* 2.35 (s, 3H), 2.41 (s, 3H), 3.16 (m, 4H), 3.25 (m, 4H), 6.89 (m, 1 H), 6.91-6.94 (m, 2H), 7.06 (m, 1 H), 7.08 (m, 1 H), 7.26-7.30 (m, 2H), 7.53 (d, J = 7.8 Hz, 1 H); MS (ESI) m/z: 299 [MH]⁺.

### Example 9: Preparation of 1-((2,4-dimethylphenyl)thio)-4-phenylpiperazine (Ih)

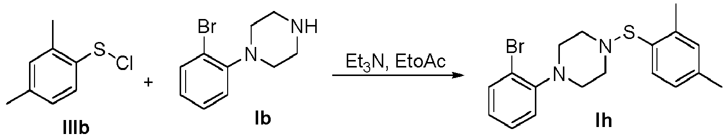

To a solution of **Ib** (0.82 g, 3.41 mmol) and Et₃N (0.60 mL, 4.31 mmol) in EtOAc (5 mL) was slowly added **IIIb** (0.62 g, 3.59 mmol) while stirring at room temperature. After stirring for 1h reaction mixture was concentrated, heptane (10 mL) was added, salts were filtered off and washed with heptane (2 x 10 mL), and filtrate was concentrated to afford title compound as yellow oil, which crystalized upon standing (1.07 g, 83% yield): ¹H NMR (CDCl₃, 500 MHz) *δ* 2.34 (s, 3H), 2.39 (s, 3H), 3.11 (m, 4H), 3.19 (m, 4H), 6.93 (m, 1 H), 7.04-7.08 (m, 3H), 7.28 (m, 1 H), 7.53 (d, *J* = 7.6 Hz, 1 H), 7.55 (dd, *J* = 7.6, 1.4 Hz, 1 H); MS (ESI) *m*/*z*: 377 [MH]⁺.

### 1-((2,4-Dimethylphenyl)thio)-4-phenylpiperazine (Ih) was also prepared directly from 2,4-dimethylbenzenethiol:

To a solution of trichloroisocyanuric acid (2.24 g, 9.65 mmol) in EtOAc (50 mL) was slowly added 2,4-dimethylbenzenethiol (0.49 mL, 3.62 mmol). Obtained reaction mixture was stirred at room temperature for 30 min, then Et₃N (10.1 mL, 72.3 mmol) and solution of **Ib** (6.62 g, 27.5 mmol) in EtOAc (50 mL) was added. After stirring at room temperature for 2 h, salts were filtered off, washed with EtOAc (2 x 20 mL), and filtrate was concentrated. To the remaining MeOH (100 mL) was added and the obtained mixture was stirred at room temperature for 1 h. White precipitate was filtered off, washed with MeOH (2 x 20 mL) and dried to afford title compound as white powder (8.36 g, 81% yield): ¹H NMR (CDCl₃, 500 MHz) *δ* 2.34 (s, 3H), 2.39 (s, 3H), 3.11 (m, 4H), 3.19 (m, 4H), 6.93 (m, 1 H), 7.04-7.08 (m, 3H), 7.28 (m, 1H), 7.53 (d, *J* = 7.6 Hz, 1 H), 7.55 (dd, J = 7.6, 1.4 Hz, 1 H); MS (ESI) m/z: 377 [MH]⁺.

### Example 10: Preparation of 1-(2-((2,4-dimethylphenyl)thio)phenyl)piperazine (vortioxetine, IVa) from 1-((2,4-dimethylphenyl)thio)-4-phenylpiperazine (Ih)

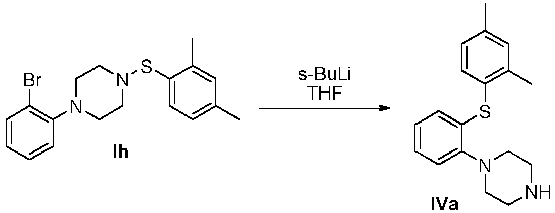

To a solution of **Ih** (0.5 g, 1.48 mmol) in dry THF (10 mL) was added sBuLi (1.48 mmol) while stirring at -80°C. Obtained reaction mixture was stirred at -80°C for 15 min then reaction temperature was slowly increased to room temperature. After stirring at room temperature for 1 h, water (2 mL) and EtOAc (10 mL) were added. Organic phase was then washed with water (2 x 25 mL) and brine (2 x 25 mL), dried over Na₂SO₄ and concentrated to afford crude product, which is then purified by chromatography to afford title compound (0.22 g, 50% yield): ¹H NMR (CDCl₃, 500 MHz) *δ* 1.63 (br s, 1 H), 2.33 (s, 3H), 2.37 (s, 3H), 3.02-3.09 (m, 8H), 6.52 (m, 1 H), 6.87 (m, 1H), 7.04 (m, 1 H), 7.06-7.10 (m, 2H), 7.16 (m, 1 H), 7.39 (d, *J* = 7.8 Hz, 1 H); MS (ESI) *m*/*z*: 299 [MH]⁺.

### Example 11: Preparation of 1-(2-((2,4-dimethylphenyl)thio)phenyl)piperazine hydrochloride (IVa.HCl)

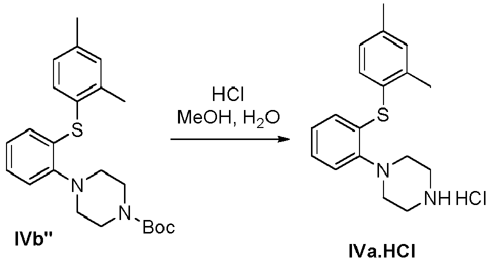

A mixture of **IVb"** (1.78 g, 4.47 mmol), MeOH (10 mL) water (2 mL), and 37% HCl (6 mL) was stirred at room temperature for 3 days. To the reaction mixture water (30 mL) was added, precipitate was filtered off. After washing with water (10 mL) and MeOAc (2 x 20 mL) it was dried to afford title compound as HCl salt. Off white powder (1.27 g, 85% yield): DSC: Onset 230.31°C, Peak 323.15°C.

### Example 12: Preparation of 1-(2-((2,4-dimethylphenyl)thio)phenyl)piperazine hydrobromide (vortioxetine HBr, IVa.HBr)

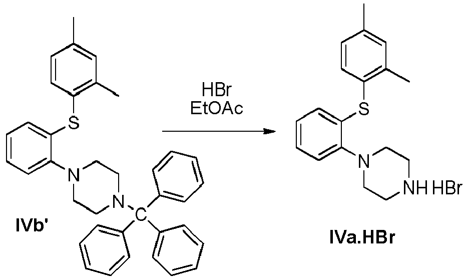

A solution of **IVb'** (1.0 g, 1.85 mmol) in EtOAc (25 mL) 48% HBr (0.5 mL) was added. Obtained reaction mixture was stirred at room temperature for 6 h. Precipitate was filtered off, washed with EtOAc (2 x 10 mL), and dried to afford title compound as a beige powder (0.41 g, 59% yield): ¹H NMR (DMSO-*d₆*, 500 MHz) *δ* 2.23 (s, 3H), 2.32 (s, 3H), 3.15-3.27 (m, 8H), 6.40 (m, 1 H), 6.96 (m, 1 H), 7.08-7.17 (m, 3H), 7.24 (m, 1 H), 7.32 (d, *J* = 7.8 Hz, 1 H), 8.85 (br, 2H).

### Example 13: Preparation of 1-(2-((2,4-dimethylphenyl)thio)phenyl)piperazine hydrobromide (vortioxetine HBr, IVa.HBr)

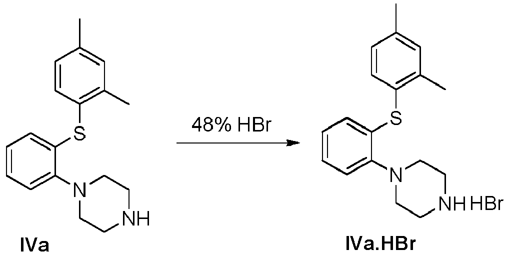

To a solution of vortioxetine **IVa** (1.80 g, 6.03 mmol) in iPrOAc (20 mL) at room temperature 48% HBr (0.68 mL, 6.03 mmol) was slowly added. Obtained mixture was stirred at room temperature for 1 h, white precipitate was then filtered off, washed with acetone (2 x 20 mL), and dried to afford title compound as white powder (2.15 g, 94% yield): DSC: Onset 221.74°C, Peak 223.86°C; ¹H NMR (DMSO-*d₆*, 500 MHz) *δ* 2.23 (s, 3H), 2.32 (s, 3H), 3.15-3.27 (m, 8H), 6.40 (m, 1 H), 6.96 (m, 1 H), 7.08-7.17 (m, 3H), 7.24 (m, 1 H), 7.32 (d, *J* = 7.8 Hz, 1 H), 8.85 (br, 2H).

### Reference Example: Preparation of 2,4-dimethylphenyl hypochlorothioite (IIIb)

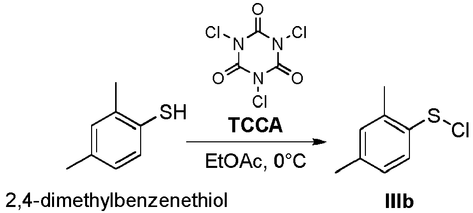

To a solution of trichloroisocyanuric acid **TCCA** (0.28 g, 1.21 mmol) in EtOAc (5 mL) was slowly added 2,4-dimethylbenzenethiol (0.49 mL, 3.62 mmol) at 0°C. After stirring at 0°C for 1 h EtOAc was evaporated, heptane (10 mL) was added, white precipitate was filtered off, washed with heptane (2 x 10 mL), and filtrate was evaporated to afford title compound **IIIb** as an orange oil (0.62 g, 99% yield): ¹H NMR (CDCl₃, 500 MHz) *δ* 2.37 (s, 3H), 2.60 (s, 3H), 7.05 (m, 1H), 7.16 (m, 1 H), 7.66 (d, *J* = 7.9 Hz, 1 H).

## Claims

1. A process for the manufacture of a compound of formula **IVa,** or a salt thereof, 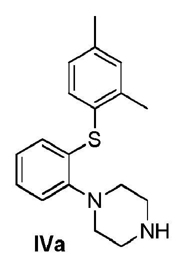 said process comprising the steps of
a) reacting a compound of formula I 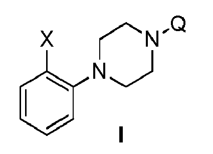 wherein X represents hydrogen or a halogen, and Q represents hydrogen, an amino protecting group, or the 2,4-dimethylphenylsulfide moiety of formula 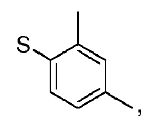 which 2,4-dimethylphenylsulfide moiety is coupled to the nitrogen of formula **I** via the sulphur atom,
by lithiation, to give a compound of formula **II** or **II'** 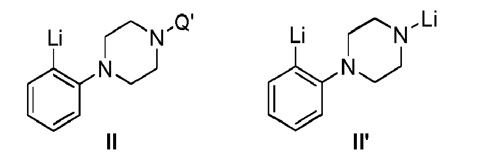 wherein Q' represents said amino protecting group, or the 2,4-dimethylphenylsulfide moiety, and
b) converting the compound of formula **II** or **II'** into the compound of formula **IVa,**
c) optionally, converting the obtained compound of formula **IVa** into the salt thereof.

2. The process according to claim 1, wherein Q in the compound of formula **I** represents hydrogen, and step b) is conducted by introducing the 2,4-dimethylphenylsulfide moiety to the compound of formula **II**' to give a compound of formula **IVa.**

3. The process according to claim 1, wherein Q in the compound of formula **I** represents an amino protecting group, and step b) is conducted by introducing the 2,4-dimethylphenylsulfide moiety to the compound of formula **II** to give a compound of formula **IVb** 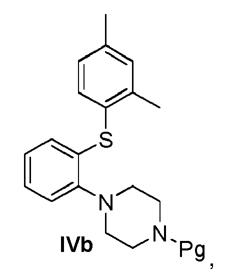 and, deprotecting the protected amino group to obtain the compound of formula **IVa.**

4. The process according to claim 2 or claim 3, wherein the 2,4-dimethylphenylsulfide moiety is introduced using 1,2-bis(2,4-dimethylphenyl)disulfane or 2,4-dimethylphenylsulfenyl halide, preferably 2,4-dimethylphenyl hypochlorothioite.

5. The process according to claim 1, wherein Q in the compound of formula **I** represents the 2,4-dimethylphenylsulfide moiety of formula 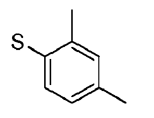 wherein said moiety is coupled to the nitrogen of formula **I** via the sulphur atom, and
step b) is conducted by the re-arrangement of the 2,4-dimethylphenylsulfide moiety to give the compound of formula **IVa.**

6. The process according to claim 5, wherein the lithiation temperature is from -100°C to -50°C, and the re-arrangement of the 2,4-dimethylphenylsulfide moiety is conducted at a temperature of from 0°C to 50°C.

7. The process according to claim 5 or claim 6, wherein the compound of formula **I**, wherein Q represents the 2,4-dimethylphenylsulfide moiety of formula 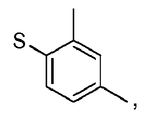 is prepared by a process comprising the steps of
a) reacting 2,4-dimethylbenzenethiol with trichloroisocyanuric acid to give 2,4-dimethylphenyl hypochlorothioite, and
b) reacting the 2,4-dimethylphenyl hypochlorothioite with the compound of formula **I"** 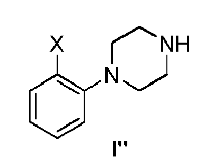 wherein X represents hydrogen or halogen.

8. The process according to claim 1, wherein the compound of formula **II** or **II'** is not isolated.

9. The process according to any one of the previous claims, wherein the lithiation is conducted with n-, s- or t-butyllithium in an aprotic solvent.

10. The process according to claim 9, wherein the aprotic solvent is an ether or an alkane.

11. The process according to any one of the previous claims, wherein the salt of the compound of formula **IVa** is a hydrobromide salt or a hydrochloride salt.

12. A compound of formula **I'** 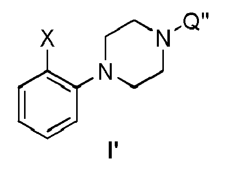 wherein X represents hydrogen or a halogen and Q" represents triphenylmethyl or 2,4-dimethylphenylsulfide.

13. A compound of formula **IVb'**

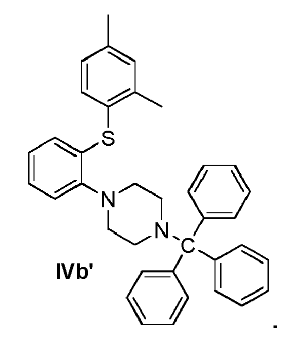

14. Use of the compounds as defined in claim 12 or claim 13 in the preparation of vortioxetine or a salt thereof.

15. A process for the preparation of a pharmaceutical composition comprising a compound of formula **IVa,** or a salt thereof 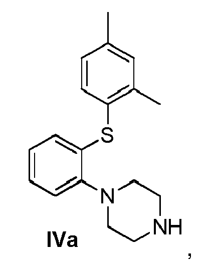 comprising the steps of:
a) preparing a compound of formula **IVa,** or a salt thereof, by carrying out a process according to any one of claims 1 to 11, and
b) mixing the compound of formula **IVa,** or a salt thereof, with a pharmaceutically acceptable carrier and/or excipient.
